# FASCICULE DE BREVET EUROPEEN

(11) **EP 1 716 826 B1**
(45) Date de publication et mention de la délivrance du brevet: **05.03.2008**
(21) Numéro de dépôt: 06356016.3
(22) Date de dépôt: 15.02.2006
(51) Int. Cl.: A61F 5/01, A61F 13/62

(54) **Orthèse réversible**
Beidseitig tragbare Orthese
Reversible orthosis

(30) Priorité: 15.02.2005 FR 0501507
(43) Date de publication de la demande: 02.11.2006
(73) Titulaire: RICHARD FRERES S.A., 42530 Saint Genest Lerpt (FR)
(72) Inventeur: Richard, Dominique, 42110 Aurec/Loire (FR)
(74) Mandataire: Delorme, Nicolas

(56) Documents cités:
- EP-A- 0 761 184
- GB-A- 2 181 952
- US-A- 4 550 724
- US-A- 5 928 172

## Description

La présente invention concerne une orthèse réversible, du type comportant une pièce textile présentant une première et une deuxième faces opposées et destinée à être placée autour d'une partie du corps d'un utilisateur pour soutenir et/ou immobiliser cette partie du corps.

La réversibilité d'une orthèse peut être rendue possible notamment par le fait qu'elle est dépourvue d'envers et peut donc être portée indifféremment avec sa première ou sa seconde face en contact du corps d'un utilisateur. Ainsi, une orthèse réversible peut être utilisée indifféremment pour une même partie gauche ou droite du corps de l'utilisateur.

L'invention se rapporte notamment à un gilet orthopédique de soutien et de contention de l'épaule, mais peut s'appliquer à d'autres orthèses telles que des orthèses de poignet par exemple.

On connaît déjà des gilets orthopédiques réversibles, qui sont placés autour du thorax, de l'épaule et du bras d'un utilisateur et maintenus en position par des systèmes d'accrochage agrippant à boucles et crochets du type VELCRO® .

Un tel gilet est par exemple décrit dans le document US 4 550 724. Pour que ce gilet puisse effectivement être réversible, il est nécessaire de munir chacune des faces de la pièce textile de bandes agrippantes. Ceci augmente le coût du gilet, mais présente également un autre problème. En effet, lorsque le gilet équipe un utilisateur, une paire de bandes boucles - crochets est inutilisée, la paire inutilisée dépendant de l'épaule - gauche ou droite - équipée. Il s'ensuit que, d'une part, la première des bandes inutilisées peut être en contact avec la peau de l'utilisateur, ce qui nuit au confort de ce dernier et, d'autre part, la deuxième bande inutilisée est exposée en face extérieure du gilet, et peut de ce fait s'accrocher à différents objets ou matériaux.

Pour pallier cet inconvénient, il peut être prévu un rabat muni d'une bande complémentaire de la bande inutilisée et destinée à coopérer avec elle. Cette solution nécessite donc un élément supplémentaire, ce qui présente un certain nombre de désavantages. Tout d'abord, l'augmentation de la quantité de textile et de bandes de textile agrippant de type VELCRO® utilisée induit une augmentation du coût du gilet. De plus, le rabat rigidifie le gilet qui, de ce fait, s'avère moins efficace et moins confortable car plus difficilement adaptable à l'anatomie de l'utilisateur. Enfin, du fait de la surépaisseur créée, l'esthétique du gilet se trouve altérée.

La présente invention, qui est définie par la revendication 1, vise à remédier aux inconvénients mentionnés ci-dessus.

A cet effet, l'invention concerne une orthèse orthopédique pouvant maintenir et/ou immobiliser au moins un membre d'un patient, comportant une pièce textile présentant une première et une deuxième faces, lesdites première face et deuxième face pouvant indifféremment venir en contact du ou des membres du patient.

L'orthèse est plus particulièrement caractérisée en ce que l'orthèse présente, en outre, au moins une patte d'accrochage comportant une face active présentant des éléments d'accrochage aptes à coopérer avec des premiers ou des deuxièmes moyens d'attache disposés respectivement sur la première face et la deuxième face de la pièce textile, et une face inactive opposée à la face active, ladite patte d'accrochage étant fixée, de façon inamovible, par sa face inactive sur une zone de la pièce textile contiguë à l'un des bords de ladite pièce textile de façon à pouvoir pivoter autour d'un axe de pivotement sensiblement parallèle à ce bord entre une première position dans laquelle la face active vient s'agripper par contact sur les premiers moyens d'attache et une deuxième position dans laquelle la face active vient s'agripper par contact sur les deuxième moyens de d'attache.

De cette façon, la face active de la patte peut être orientée selon les besoins, afin d'être placée en regard de la surface sur laquelle elle doit être accrochée. Ainsi, l'orthèse est totalement adaptable à la partie gauche ou droite du corps humain, de par la forme et la réversibilité de la pièce textile mais également de par la capacité de pivotement des moyens d'accrochage. La patte forme en quelque sorte le prolongement de la première ou de la deuxième face selon le sens d'utilisation de la pièce textile.

La patte peut comprendre une première et une deuxième ailes sensiblement identiques s'étendant de part et d'autre de l'axe de pivotement, la première aile étant apte à être rabattue contre la première face de la pièce textile, dans la première position de la patte, et la deuxième aile étant apte à être rabattue contre la deuxième face de la pièce textile, dans la deuxième position de la patte.

Toute la surface active de la patte est utilisée, et ce quel que soit le sens dans lequel est disposée la pièce textile, c'est-à-dire quelle que soit l'orientation de la patte.

Selon un premier mode de réalisation, la patte est fixée sur une bande de la pièce textile délimitée d'un côté par le bord de la pièce textile et de l'autre par l'axe de pivotement de la patte. Ce mode de fixation est particulièrement solide.

Selon un deuxième mode de réalisation, la patte est fixée sensiblement sur le bord de la pièce textile, ledit bord formant l'axe de pivotement de la patte. On obtient ainsi une structure parfaitement symétrique, sans surépaisseur, et un pivotement aisé de la patte.

La patte peut être fixée sur la pièce textile par couture, soudage ou collage.

Selon une réalisation possible, la patte s'étend sur sensiblement toute la longueur du bord de la pièce textile. En variante, on pourrait prévoir le long du bord plusieurs bandes espacées les unes des autres.

Par exemple, les éléments d'accrochage sont constitués par des crochets et les premiers et deuxièmes moyens d'attache disposés respectivement sur la première face et la deuxième face de la pièce textile sont constitués par des boucles, formant un système de fixation auto agrippant du type VELCRO® . Cette disposition s'avère particulièrement confortable pour le patient puisqu'il a ainsi en contact de sa peau un textile à boucles dont le contact est agréable et doux.

Les premiers et/ou deuxièmes moyens d'attache comprennent une bande rapportée sur la pièce textile, ou sont constitués par le textile même de la pièce textile ; cette dernière disposition s'avère extrêmement pratique puisqu'elle n'induit pas de surépaisseur.

Selon une réalisation possible, la pièce textile est destinée à former un gilet orthopédique de soutien et de contention de l'épaule, et comprend :
- un premier panneau destiné à recouvrir la partie arrière du thorax de l'utilisateur ;
- un deuxième panneau prolongeant latéralement le premier panneau et destiné à recouvrir la partie avant et la partie arrière de l'épaule et du bras ;
- un troisième panneau prolongeant latéralement le deuxième panneau, à l'opposé du premier panneau, et destiné à recouvrir la partie avant du thorax ;
- un quatrième panneau prolongeant partiellement la partie inférieure du troisième panneau et destiné à servir de reposoir à l'avant-bras.

Au moins une patte d'accrochage peut être fixée au voisinage du bord du premier panneau opposé au deuxième panneau et/ou du bord du quatrième panneau opposé au troisième panneau, ladite patte étant destinée à coopérer avec des moyens d'attache ménagés sur le troisième panneau.

L'orthèse peut en outre comprendre une ceinture s'étendant latéralement depuis la partie inférieure du troisième panneau dans le même sens que le deuxième panneau, et une patte d'accrochage fixée au voisinage du bord de la ceinture opposé au troisième panneau, ladite patte étant destinée à coopérer avec des moyens d'attache ménagés sur le premier panneau.

Pour sa bonne compréhension, l'invention est à nouveau décrite ci-dessous en référence aux figures annexées représentant, à titre d'exemples non limitatifs, plusieurs formes de réalisation possibles d'orthèses selon celle-ci.

La figure 1 est une vue en plan d'une pièce textile destinée à former un gilet orthopédique, un coin de la pièce textile ayant été recourbé pour une meilleure compréhension ;

La figure 2 est une vue en perspective d'une sangle destinée à être associée à la pièce textile de la figure 1 ;

La figure 3 est une vue agrandie du détail III de la figure 1, montrant la patte d'accrochage, selon un premier mode de réalisation ;

La figure 4 est une vue similaire à la figure 3, la patte d'accrochage ayant pivoté par rapport à son axe de pivotement ;

Les figures 5 et 6 sont des représentations schématiques de la liaison entre la patte et la pièce textile, respectivement selon le premier et selon un deuxième mode de réalisation ;

Les figures 7 et 8 représentent les étapes de mise en place du gilet de la figure 1 ; et

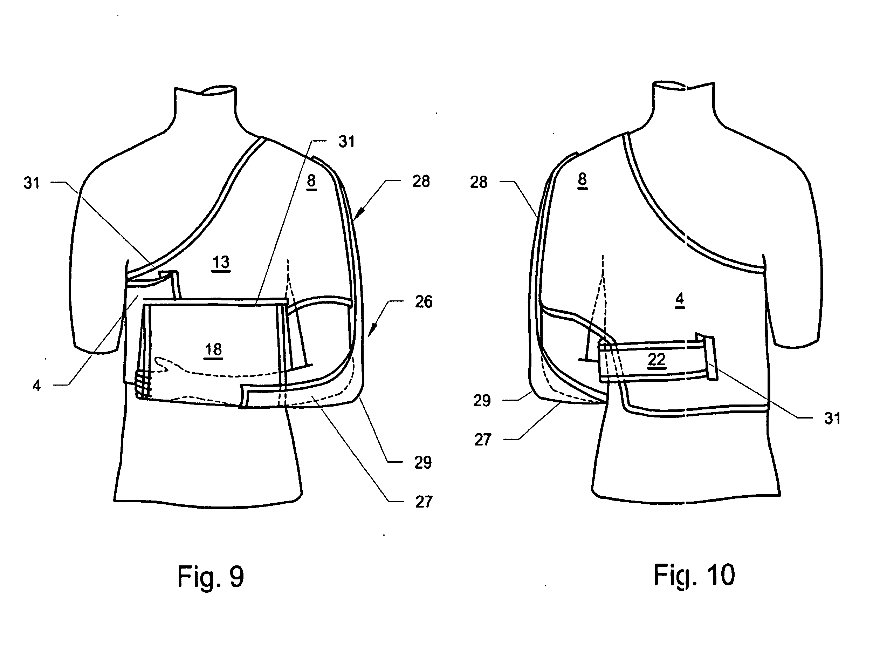

Les figures 9 et 10 représentent schématiquement un utilisateur équipé du gilet de la figure 1, respectivement de face et de dos.

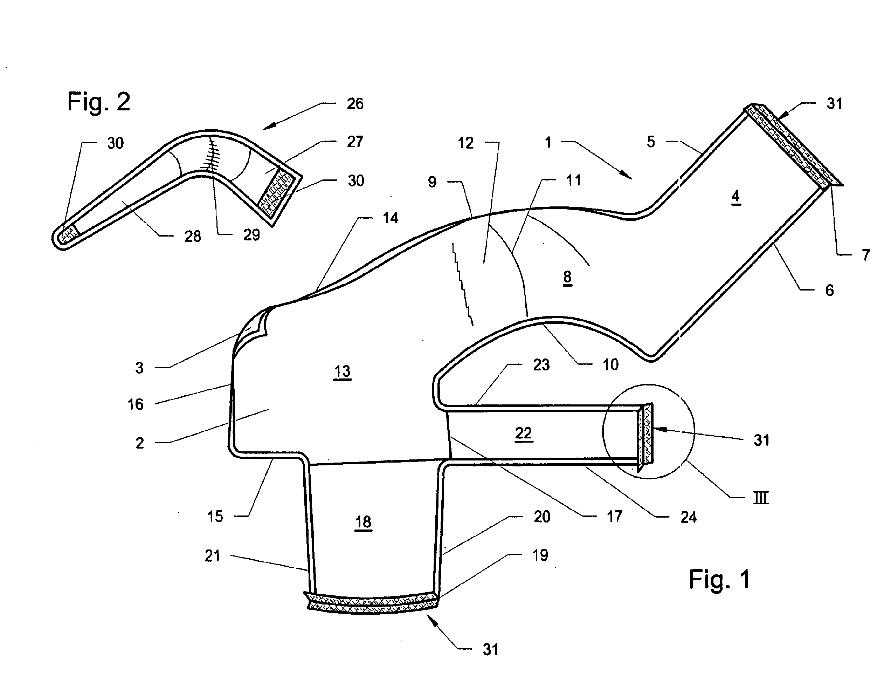

La figure 1 représente une pièce textile 1, vue en plan, qui, une fois mise en place sur un utilisateur, formera un gilet orthopédique.

La pièce textile 1 est globalement plane et présente une première face 2 et une deuxième face 3, opposée, visible partiellement dans le coin représenté à cet effet de façon partiellement rabattue.

La pièce textile 1 comprend tout d'abord un premier panneau 4, sensiblement rectangulaire, destiné à recouvrir la partie arrière du thorax d'un utilisateur, délimité notamment par un bord supérieur 5, un bord inférieur 6 et un bord libre 7.

La pièce textile 1 comprend également un deuxième panneau 8 prolongeant latéralement le premier panneau 4 à l'opposé du bord libre 7, et destiné à recouvrir la partie avant et la partie arrière de l'épaule et du bras. Le deuxième panneau 8 forme un coude incurvé à sa partie inférieure, et est délimité notamment par un bord supérieur. 9 et un bord inférieur 10 courbes. En outre, une ligne courbe 11 s'étendant entre les bords supérieur 9 et inférieur 10, sensiblement au milieu du deuxième panneau 8, forme un bossage 12 localisé en partie supérieure du deuxième panneau 8, de façon sensiblement centrée latéralement. Sur la figure 1, le bossage 12 fait saillie vers le haut par rapport au plan général de la pièce textile 1. La concavité du bossage 12 peut être inversée, afin que le bossage 12 puisse faire saillie vers le bas par rapport au plan général de la pièce textile 1. C'est notamment grâce à cette inversion de concavité que le gilet peut être adapté à l'épaule gauche ou droite d'un utilisateur.

Un troisième panneau 13, sensiblement rectangulaire, prolonge latéralement le deuxième panneau 8 à l'opposé du premier panneau 4, et est destiné à recouvrir la partie avant du thorax de l'utilisateur. Le troisième panneau 13 est délimité, notamment, par un bord supérieur 14, un bord inférieur 15 et un bord libre 16. La distance entre les bords supérieur 14 et inférieur 15 du troisième panneau 13 est supérieure à la distance entre les bords supérieur 9 et inférieur 10 du deuxième panneau 8. Le troisième panneau 13 comprend ainsi un bord latéral 17, de faible hauteur, opposé au bord libre 16, et non lié au deuxième panneau 8.

Un quatrième panneau 18 prolonge partiellement le troisième panneau 13 à sa partie inférieure, et est destiné à servir de reposoir à l'avant-bras de l'utilisateur. Le quatrième panneau 18 présente la forme générale d'un trapèze dont les bases forment les bords supérieur et inférieur (libre) 19 du quatrième panneau 18, le bord supérieur étant confondu avec le bord inférieur 15 du troisième panneau 13. Le quatrième panneau 18 est en outre délimité par un bord latéral 20 situé sensiblement en prolongement du bord latéral 17 du troisième panneau 13 et un bord latéral 21. La distance entre les bords latéraux 20, 21 du quatrième panneau 18 est inférieure (par exemple de l'ordre des deux tiers) à la distance entre les bords libre 16 et latéral 17 du troisième panneau 13.

Enfin, la pièce textile 1 comporte une ceinture 22 s'étendant latéralement depuis le bord 17 du troisième panneau 13 vers et sensiblement jusqu'au premier panneau 4, sensiblement parallèlement au bord inférieur 15 du troisième panneau 13. La ceinture présente un bord supérieur 23 et un bord inférieur 24 ainsi qu'un bord libre 25 opposé au bord latéral 17.

Les première et deuxième faces 2, 3 sont réalisées en un matériau présentant des boucles, à la manière des boucles d'une fixation de type VELCRO ® . Par exemple, la pièce textile 1 peut être réalisée en mousse polyuréthanne recouverte d'une duvetine à boucles en polyamide. En variante, les première et deuxième faces 2, 3 pourraient ne pas présenter de boucles mais pourraient être munies de bandes rapportées qui, elles, présenteraient des boucles.

La figure 2 représente une sangle 26 comprenant un premier panneau 27 rectangulaire et un deuxième panneau 28 sensiblement rectangulaire effilé en pointe, associés par une ligne transversale courbe 29 conférant à la sangle une forme de coude. Le premier panneau 27 présente, à ses extrémités et d'un même côté, deux zones 30 munies d'éléments d'accrochage du type crochets d'un système VELCRO®.

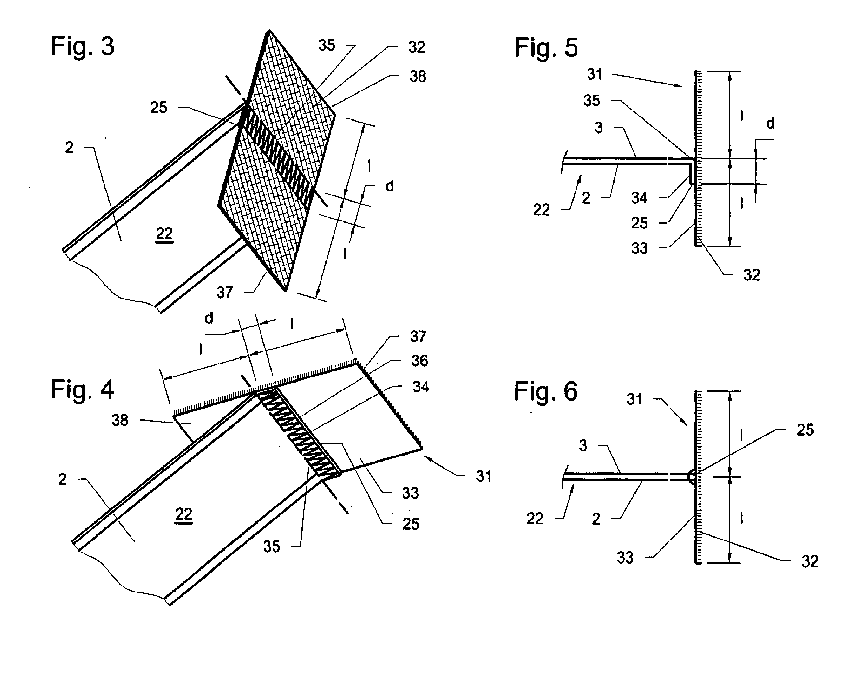

De façon spécifique à l'invention, les bords libres 7 du premier panneau 4, 19 du quatrième panneau 18 et 25 de la ceinture 22 de la pièce textile 1 comportent chacun une patte d'accrochage 31 s'étendant sur toute la longueur dudit bord. La patte d'accrochage 31 liée à la ceinture 22 va maintenant être décrite plus précisément en référence aux figures 3 à 6, étant entendu que les autres pattes et leur mode de fixation sont similaires.

La patte 31 est rectangulaire plane et présente une face active 32 comprenant des éléments d'accrochage du type crochets d'un système VELCRO ® . La face opposée, dite face inactive 33, est dépourvue de tels éléments d'accrochage. La patte 31 est fixée par sa face inactive 33 à la pièce textile 1, ici la ceinture 22, au voisinage du bord considéré, ici le bord libre 25.

Selon un premier mode de réalisation, représenté sur les figures 3 à 5, la patte 31 est fixée sur une bande 34 de la ceinture 22, de largeur d, limitée d'une part par le bord 25, et d'autre part par une ligne 35 parallèle au bord 25. La fixation est ici réalisée par une couture 36 en zigzag. Une couture droite pourrait être envisagée.

La bande 34 n'est pas fixée à la patte 31 de façon centrée, mais de sorte que la ligne 35 soit située sensiblement au milieu de la patte 31, et définisse ainsi deux ailes 37, 38 de même largeur ℓ . Du fait de la souplesse de la pièce textile 1 et de l'agencement de la couture 36, la patte 31 est apte à pivoter autour de la ligne 35, comme représenté sur les figures 3 et 4. Dans une première position extrême, la patte 31 est disposée de telle sorte que la face inactive 33 de la première aile 37 soit rabattue contre la première face 2 de la pièce textile 1, et dans une deuxième position extrême, la patte 31 est disposée de telle sorte que la face inactive 33 de sa deuxième aile 38 soit rabattue contre la deuxième face 3 de la pièce textile 1.

Ainsi, tout ce passe comme si les éléments d'accrochage de la face active 32 pouvaient être déplacés de la première face 2 à la deuxième face 3 de la pièce textile 1 selon les besoins, c'est-à-dire selon que la face de la pièce textile 1 que l'on souhaite attacher est la première ou la deuxième face, et ce par simple pivotement de la patte 31. Il n'est donc pas nécessaire de prévoir de moyens d'accrochage sur chacune des faces 2, 3 de la pièce textile. En outre, lorsque la patte 31 est dans la première position extrême, la deuxième face 3 de la pièce textile 1 est dépourvue de tout moyen d'accrochage (et inversement dans la deuxième position extrême), ce qui évite les risques d'accrochage intempestifs.

Selon un deuxième mode de réalisation, représenté sur la figure 6, la patte 31 est fixée au bord 25 de la ceinture 22, par des points boutons. Dans ce cas, le bord 25 est situé au milieu de la patte 31, définissant ainsi deux ailes 37, 38 de même largeur ℓ , et la patte 31 est apte à pivoter autour du bord 25.

La patte 31 peut être fixée sur une zone de la pièce textile 1 comportant des boucles, soit « à plat », la totalité de la surface active 32 étant en contact avec une même face de la pièce textile 1, soit « à cheval », les faces actives 32 des deux ailes 37, 38 étant rabattues l'une vers l'autre et enserrant entre elles la pièce textile 1.

On décrit à présent la mise en place de la pièce textile sur un utilisateur.

Dans un premier temps, la concavité du bossage 12 est orientée dans le sens adapté à l'épaule traumatisée à immobiliser. Sur les figures, l'épaule considérée est l'épaule gauche, et le bossage 12 doit être orienté comme représenté sur la figure 1. La face de la pièce textile 1 qui sera orientée vers l'utilisateur (à l'intérieur du gilet) est alors la deuxième face 3.

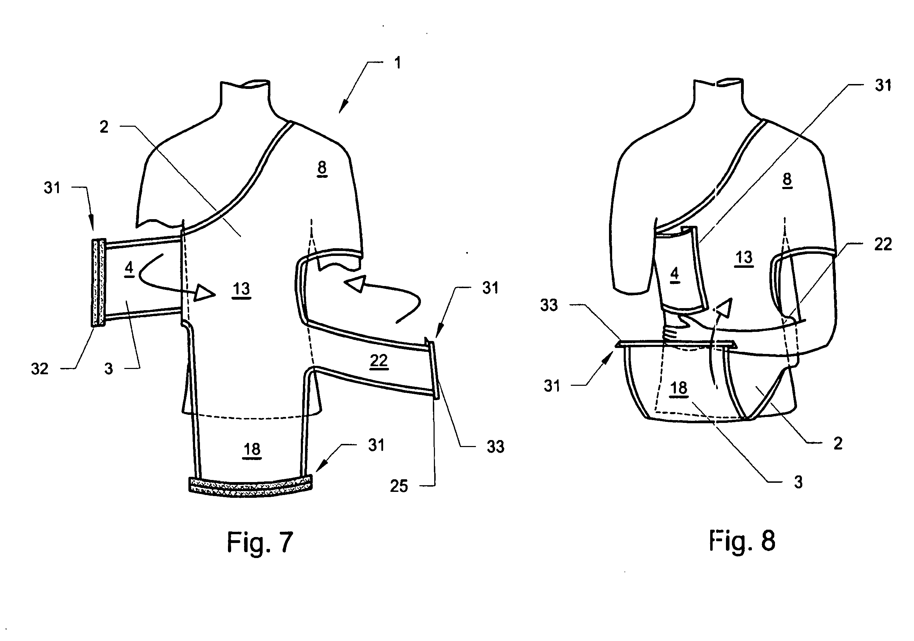

Dans un deuxième temps, le bossage 12 est placé sur l'épaule, le troisième panneau 13 couvrant l'avant du thorax, le quatrième panneau 18 descendant sur les cuisses, et le premier panneau 4 couvrant le dos (figure 7). Le premier panneau 4 est enroulé autour de l'utilisateur et rabattu contre le troisième panneau 13 sur lequel il est fixé, par coopération entre les crochets de la patte 31 et les boucles du textile du troisième panneau 13. A cet effet, la patte 31 est orientée pour que sa face active 32 soit en regard du troisième panneau 13.

De façon similaire, la ceinture 22 est enroulée autour de la taille de l'utilisateur et accrochée, dans le dos, au premier panneau 4.

L'utilisateur replie alors le bras correspondant à l'épaule traumatisée contre son ventre, puis le quatrième panneau 18 est rabattu vers le troisième panneau 13, en entourant le bras (figure 8), et accroché par la patte 31 au troisième panneau 13.

L'utilisateur peut alors fixer la sangle 26 autour de son coude, celle-ci étant fixée par les zones 30 d'une part sous le quatrième panneau 18, sous l'avant-bras, et d'autre part sur le deuxième panneau 8, sur l'épaule.

Pour la mise en place de la même pièce textile 1 sur l'autre épaule, il suffit tout d'abord d'inverser la concavité du bossage 12, puis de placer la pièce textile de façon symétrique à ce qui a été décrit précédemment (la deuxième face 3 étant alors orientée vers l'extérieur du gilet, et partiellement apparente), et de modifier l'orientation des pattes d'accrochage 31 par simple pivotement autour de leur axe de pivotement.

Ainsi, l'invention apporte une amélioration déterminante à la technique antérieure, en fournissant une orthèse réversible dont les moyens d'accroche sont eux-mêmes réversibles.

Il va de soi que l'invention n'est pas limitée à la forme de réalisation décrite ci-dessus à titre d'exemple mais qu'elle en embrasse au contraire toutes les variantes de réalisation.

L'invention pourrait ainsi être employée pour des orthèses autres que des orthèses d'épaules (pour les poignets notamment).

## Revendications

1. Orthèse orthopédique pouvant maintenir et/ou immobiliser au moins un membre d'un patient, comportant une pièce textile (1) présentant une première et une deuxième faces (2, 3), lesdites première face (2) et deuxième face (3) pouvant indifféremment venir en contact du ou des membres du patient des premiers et des deuxièmes moyens d'attache étant disposés respectivement sur la première face (2) et la deuxième face (3) de la pièce textile (1), **caractérisée en ce que** l'orthèse présente, en outre, au moins une patte d'accrochage (31) comportant (i) une face active (32) présentant des éléments d'accrochage aptes à coopérer avec des premiers ou des deuxièmes moyens d'attache et (ii) une face inactive (33) opposée à la face active (32), ladite patte d'accrochage (31) étant fixée, de façon inamovible, par sa face inactive (33) sur une zone (7, 19, 25, 34) de la pièce textile (1) contiguë à l'un des bords (7, 19, 25) de ladite pièce textile (1) de façon à pouvoir pivoter autour d'un axe de pivotement (7, 19, 25, 35) sensiblement parallèle à ce bord (7, 19, 25) entre une première position dans laquelle la face active (32) vient s'agripper par contact sur les premiers moyens d'attache et une deuxième position dans laquelle la face active (32) vient s'agripper par contact sur les deuxièmes moyens de d'attache.

2. Orthèse selon la revendication 1, **caractérisée en ce que** la patte (31) comprend une première et une deuxième ailes (37, 38) sensiblement identiques s'étendant de part et d'autre de l'axe de pivotement (7, 19, 25, 35), la première aile (37) étant apte à être rabattue contre la première face (2) de la pièce textile (1), dans la première position de la patte (31), et la deuxième aile (38) étant apte à être rabattue contre la deuxième face (3) de la pièce textile (1), dans la deuxième position de la patte (31).

3. Orthèse selon la revendication 1 ou 2, **caractérisée en ce que** la patte (31) est fixée sur une bande (34) de la pièce textile (1) délimitée d'un côté par le bord (7, 19, 25) de la pièce textile (1) et de l'autre par l'axe de pivotement (35) de la patte (31).

4. Orthèse selon la revendication 1 ou 2, **caractérisée en ce que** la patte (31) est fixée sensiblement sur le bord (7, 19, 25) de la pièce textile (1), ledit bord (7, 19, 25) formant l'axe de pivotement de la patte (31).

5. Orthèse selon l'une des revendications 1 à 4, **caractérisée en ce que** la patte (31) est fixée sur la pièce textile (1) par couture, soudage ou collage.

6. Orthèse selon l'une des revendications 1 à 5, **caractérisée en ce que** la patte (31) s'étend sur sensiblement toute la longueur du bord (7, 19, 25) de la pièce textile (1).

7. Orthèse selon l'une des revendications 1 à 6, **caractérisée en ce que** les éléments d'accrochage sont constitués par des crochets et les premiers et deuxièmes moyens d'attache disposés respectivement sur la première face (2) et la deuxième face (3) de la pièce textile (1) sont constitués par des boucles, formant un système de fixation auto aggripant du type VELCRO^{®}.

8. Orthèse selon l'une des revendications 1 à 7, **caractérisée en ce que** les premiers et/ou deuxièmes moyens d'attache comprennent une bande rapportée sur la pièce textile (1).

9. Orthèse selon l'une des revendications 1 à 7, **caractérisée en ce que** les premiers et/ou deuxièmes moyens d'attache sont constitués dans le textile même de la pièce textile (1).

10. Orthèse selon l'une des revendications 1 à 8, **caractérisée en ce que** la pièce textile (1) est destinée à former un gilet orthopédique de soutien et de contention de l'épaule, et comprend :
un premier panneau (4) destiné à recouvrir la partie arrière du thorax de l'utilisateur ;
un deuxième panneau (8) prolongeant latéralement le premier panneau (4) et destiné à recouvrir la partie avant et la partie arrière de l'épaule et du bras ;
un troisième panneau (13) prolongeant latéralement le deuxième panneau (8), à l'opposé du premier panneau (4), et destiné à recouvrir la partie avant du thorax ;
un quatrième panneau (18) prolongeant partiellement la partie inférieure du troisième panneau (13) et destiné à servir de reposoir à l'avant-bras.

11. Orthèse selon la revendication 10, **caractérisée en ce qu**'elle comprend au moins une patte (31) d'accrochage fixée au voisinage du bord (7) du premier panneau (4) opposé au deuxième panneau (8) et/ou du bord (19) du quatrième panneau (18) opposé au troisième panneau (13), ladite patte (31) étant destinée à coopérer avec des moyens d'attache ménagés sur le troisième panneau (13).

12. Orthèse selon la revendication 10 ou 11, **caractérisée en ce qu'**elle comprend en outre une ceinture (22) s'étendant latéralement depuis la partie inférieure du troisième panneau (13) dans le même sens que le deuxième panneau (8), et une patte (31) d'accrochage fixée au voisinage du bord (25) de la ceinture (22).

## Claims

1. Orthopedic orthosis which can support and/or immobilize at least one limb of a patient, comprising a textile article (1) with first and second faces (2,3), either of which first (2) and second (3) faces can equally well come into contact with the limb or limbs of the patient, first and second securing means being arranged respectively on the first face (2) and the second face (3) of the textile article (1), **characterized in that** the orthosis additionally has at least one fastening tab (31) comprising (i) an active face (32) with fastening elements that are able to cooperate with the first or second securing means, and (ii) an inactive face (33) opposite the active face (32), said fastening tab (31) being fixed non-removably via its inactive face (33) on a zone (7, 19, 25, 34) of the textile article (1) contiguous to one of the edges (7, 19, 25) of said textile article (1), so as to be able to pivot, about a pivot axis (7, 19 ,25, 35) substantially parallel to this edge (7, 19, 25), between a first position, in which the active face (32) catches by contact on the first securing means, and a second position, in which the active face (32) catches by contact on the second securing means.

2. Orthosis according to Claim 1, **characterized in that** the tab (31) comprises substantially identical first and second wings (37, 38) extending on either side of the pivot axis (7, 19, 25, 35), the first wing (37) being able to be folded back against the first face (2) of the textile article (1), in the first position of the tab (31), and the second wing (38) being able to be folded back against the second face (3) of the textile article (1), in the second position of the tab (31).

3. Orthosis according to Claim 1 or 2, **characterized in that** the tab (31) is fixed on a band (34) of the textile article (1) delimited on one side by the edge (7, 19, 25) of the textile article (1) and on the other side by the pivot axis (35) of the tab (31).

4. Orthosis according to Claim 1 or 2, **characterized in that** the tab (31) is fixed substantially on the edge (7, 19, 25) of the textile article (1), said edge (7, 19, 25) forming the pivot axis of the tab (31).

5. Orthosis according to one of Claims 1 to 4, **characterized in that** the tab (31) is fixed on the textile article (1) by sewing, welding or bonding.

6. Orthosis according to one of Claims 1 to 5, **characterized in that** the tab (31) extends along substantially the entire length of the edge (7, 19, 25) of the textile article (1).

7. Orthosis according to one of Claims 1 to 6, **characterized in that** the fastening elements are formed by hooks, and the first and second securing means arranged respectively on the first face (2) and second face (3) of the textile article (1) are formed by loops, establishing a self-adhering fixation system of the Velcro^{®} type.

8. Orthosis according to one of Claims 1 to 7, **characterized in that** the first and/or second securing means comprise a band attached to the textile article (1).

9. Orthosis according to one of Claims 1 to 7, **characterized in that** the first and/or second securing means are formed within the actual textile of the textile article (1).

10. Orthosis according to one of Claims 1 to 8, **characterized in that** the textile article (1) is intended to form an orthopedic vest for supporting and immobilizing the shoulder, and comprises:
a first panel (4) intended to cover the rear area of the user's thorax;
a second panel (8) forming a lateral continuation of the first panel (4) and intended to cover the front area and rear area of the shoulder and of the arm;
a third panel (13) forming a lateral continuation of the second panel (8), remote from the first panel (4), and intended to cover the front area of the thorax;
a fourth panel (18) forming a continuation of a portion of the lower part of the third panel (13) and intended to serve as a rest for the forearm.

11. Orthosis according to Claim 10, **characterized in that** it comprises at least one fastening tab (31) fixed near the edge (7) of the first panel (4) remote from the second panel (8) and/or the edge (19) of the fourth panel (18) remote from the third panel (13), said tab (31) being intended to cooperate with securing means formed on the third panel (13).

12. Orthosis according to Claim 10 or 11, **characterized in that** it additionally comprises a belt (22) extending laterally from the lower part of the third panel (13) in the same direction as the second panel (8), and a fastening tab (31) fixed near the edge (25) of the belt (22).

## Patentansprüche

1. Orthopädische Orthese zum Halten und/oder Ruhigstellen zumindest eines Körperteils eines Patienten, mit einem Textilteil (1), das eine erste und eine zweite Seite (2, 3) aufweist, wobei die erste Seite (2) und die zweite Seite (3) in gleicher Weise mit dem oder den Körperteilen des Patienten in Berührung kommen können, wobei erste und zweite Befestigungsmittel entsprechend an der ersten Seite (2) und der zweiten Seite (3) des Textilteils (1) angeordnet sind, **dadurch gekennzeichnet, dass** die Orthese ferner zumindest eine Ankoppellasche (31) aufweist, die (i) eine aktive Seite (32), die Ankoppelelemente aufweist, die geeignet sind, mit den ersten oder zweiten Befestigungsmitteln zusammenzuwirken, und (ii) eine inaktive Seite (33) aufweist, die der aktiven Seite (32) gegenüber liegt, wobei die Ankoppellasche (31) mittels ihrer inaktiven Seite (33) nicht abnehmbar an einem Bereich (7, 19, 25, 34) des Textilteils (1), der zu einem der Ränder (7, 19, 25) des Textilteils (1) benachbart ist, befestigt ist, so dass sie um eine Schwenkachse (7, 19, 25, 35), die etwa parallel zu diesem Rand (7, 19, 25) ist, zwischen einer ersten Stellung, in der sich die aktive Seite (32) durch Berührung an den ersten Befestigungsmitteln festhält, und einer zweiten Stellung verschwenken kann, in der sich die aktive Seite (32) durch Berührung an den zweiten Befestigungsmitteln festhält.

2. Orthese nach Anspruch 1, **dadurch gekennzeichnet, dass** die Lasche (31) einen ersten und einen zweiten Flügel (37, 38) aufweist, die etwa identisch sind und sich beidseitig der Schwenkachse (7, 19, 25, 35) erstrecken, wobei der erste Flügel (37) geeignet ist, gegen die erste Seite (2) des Textilteils (1) in die erste Stellung der Lasche (31) umgeklappt zu werden, und wobei der zweite Flügel (38) geeignet ist, gegen die zweite Seite (3) des Textilteils (1) in die zweite Stellung der Lasche (31) umgeklappt zu werden.

3. Orthese nach Anspruch 1 oder 2, **dadurch gekennzeichnet, dass** die Lasche (31) an einem Streifen (34) des Textilteils (1) befestigt ist, der einerseits durch den Rand (7, 19, 25) des Textilteils (1) und andererseits durch die Schwenkachse (35) der Lasche (31) begrenzt ist.

4. Orthese nach Anspruch 1 oder 2, **dadurch gekennzeichnet, dass** die Lasche (31) etwa an dem Rand (7, 19, 25) des Textilteils (1) befestigt ist, wobei der Rand (7, 19, 25) die Schwenkachse der Lasche (31) bildet.

5. Orthese nach einem der Ansprüche 1 bis 4, **dadurch gekennzeichnet, dass** die Lasche (31) durch Vernähen, Löten oder Kleben an dem Textilteil (1) befestigt ist.

6. Orthese nach einem der Ansprüche 1 bis 5, **dadurch gekennzeichnet, dass** sich die Lasche (31) über etwa die gesamte Länge des Randes (7, 19, 25) des Textilteils (1) erstreckt.

7. Orthese nach einem der Ansprüche 1 bis 6, **dadurch gekennzeichnet, dass** die Ankoppelelemente durch Häkchen gebildet sind, und dass die ersten und zweiten Befestigungsmittel, die entsprechend an der ersten Seite (2) und der zweiten Seite (3) des Textilteils (1) angeordnet sind, durch Schlaufen gebildet sind, die ein selbst-festhaltendes Befestigungssystem vom Typ VELCRO^{®} bilden.

8. Orthese nach einem der Ansprüche 1 bis 7, **dadurch gekennzeichnet, dass** die ersten und/oder zweiten Befestigungsmittel einen Streifen aufweisen, der an dem Textilteil (1) angesetzt ist.

9. Orthese nach einem der Ansprüche 1 bis 7, **dadurch gekennzeichnet, dass** die ersten und/oder zweiten Befestigungsmittel in dem Textilerzeugnis des Textilteils (1) selbst ausgebildet sind.

10. Orthese nach einem der Ansprüche 1 bis 8, **dadurch gekennzeichnet, dass** das Textilteil (1) dazu bestimmt ist, eine orthopädische Schulterstütz- und -retentionsweste zu bilden, und aufweist:
eine erste Bahn (4), die dazu bestimmt ist, den hinteren Abschnitt des Brustkorbs des Benutzers zu bedecken;
eine zweite Bahn (8), die die erste Bahn (4) seitlich verlängert und dazu bestimmt ist, den vorderen Abschnitt und den hinteren Abschnitt der Schulter und des Arms zu bedecken;
eine dritte Bahn (13), die die zweite Bahn (8) gegenüberliegend zur ersten Bahn (4) seitlich verlängert und dazu bestimmt ist, den vorderen Abschnitt des Brustkorbs zu bedecken;
eine vierte Bahn (18), die den unteren Abschnitt der dritten Bahn (13) teilweise verlängert und dazu bestimmt ist, zum Stützen des Unterarms zu dienen.

11. Orthese nach Anspruch 10, **dadurch gekennzeichnet, dass** sie zumindest eine Ankoppellasche (31) aufweist, die in Nähe des Randes (7) der ersten Bahn (4), die der zweiten Bahn (8) gegenüberliegt, und/oder in Nähe des Rands (19) der vierten Bahn (18), die der dritten Bahn (13) gegenüberliegt, befestigt ist, wobei die Lasche (31) dazu bestimmt ist, mit Befestigungsmitteln zusammenzuwirken, die an der dritten Bahn (13) ausgebildet sind.

12. Orthese nach Anspruch 10 oder 11, **dadurch gekennzeichnet, dass** sie ferner einen Gürtel (22), der sich seitlich von dem unteren Abschnitt der dritten Bahn (13) aus in die gleiche Richtung wie die zweite Bahn (8) erstreckt, und eine Ankoppellasche (31) aufweist, die in Nähe des Rands (25) des Gürtels (22) befestigt ist.
